# EUROPEAN PATENT SPECIFICATION

(11) **EP 0 493 124 B1**
(45) Date of publication and mention of the grant of the patent: **18.09.1996**
(21) Application number: 91312045.7
(22) Date of filing: 24.12.1991
(51) Int. Cl.: C07D 263/48

(54) **Process for the manufacture of 2-phenyl-4,5-oxazoledione 4-phenylhydrazones**
Verfahren zur Herstellung von 2-Phenyl-4,5-Oxazoldion-4-Phenylhydrazonen
Procédé pour la préparation de phényl-2 oxazoledione-4,5 phénylhydrazones-4

(30) Priority: 27.12.1990 JP 414631/90; 05.09.1991 JP 254846/91
(43) Date of publication of application: 01.07.1992
(73) Proprietor: KUREHA CHEMICAL INDUSTRY CO., LTD., Chuo-ku Tokyo 103 (JP)
(72) Inventor: Hoshi, Hajime, Iwaki-shi, Fukushima (JP); Sunagawa, Kazuhiko, Iwaki-shi, Fukushima (JP); Watanabe, Takeo, Iwaki-shi, Fukushima (JP)
(74) Representative: Geering, Keith Edwin

(56) References cited:
- EP-A- 0 282 303
- CHEMICAL ABSTRACTS, vol. 110, no. 11, March 13, 1989, Columbus, Ohio, USA SHIDA TAKAFUMI et al. "Benzoyloxamides and herbicides containing them" page 276, column 2, abstract- no. 90 617y & Jpn. Kokai Tokkyo Koho JP 63,215,658
- CHEMICAL ABSTRACTS, vol. 98, no. 13, March 28, 1983, Columbus, Ohio, USA MOHAMED ALI E. KHALIFA et al. "Synthesis of azoloyl ketone and azoloylacetic acid derivatives: reactions of 4-ary-lazo-2-oxazolin-5-ones with active methylene compounds" page 596, column 1, abstract-no. 107 224b & Heterocycles 1983, 20(1), 45-9
- CHEMICAL ABSTRACTS, vol. 100, no. 19, May 7, 1984, Columbus, Ohio, USA KUREHA CHEMICAL INDUSTRY CO., LTD. "Triazole derivatives" page 536, column 1, abstract- no. 156 607x & Jpn. Kokai Tokkyo Koho 58,185,572, 29 Oct 1983
- CHEMICAL ABSTRACTS, vol. 59, no. 3, August 5, 1963, Columbus, Ohio, USA MUSTAFA AHMED et al. "Behavior of the hetero ring in Gamma-phenyl-Delta Beta, Gamma-butenolide derivatives toward hydrazines. Acid rearrangement of 4-phenyl-hydrazono-2-phenyloxazolin-5-one" column 2815, abstract-no. 2 815h & Can. J. Chem. 41 (7), 1813-18 (1963)
- CHEMICAL ABSTRACTS, vol. 51, no. 16, August 25, 1957, Columbus, Ohio, USA GEO. W. SAWDEY "Rearrangement of 4-arylazo-2-phenyloxa- zolin-5-ones: a new synthesis of 1H-1,2,4-triazoles" column 12079, abstract-no. 12 079a & J. Am. Chem. Soc. 79, 1955-6(1957)
- CHEMICAL ABSTRACTS, vol. 45, no. 17, September 10, 1951, Columbus, Ohio, USA V. K. KUSKOV "Oxazole derivatives" column 7565, abstract-no. 7 565e & Zhur. Obshchei Khim. 21, 152-5(1951)

## Description

### BACKGROUND OF THE INVENTION

### Field of the Invention:

The present invention relates to a process for the manufacture of 2-phenyl-4,5-oxzolinedione 4-phenylhydrazone compounds which are useful as dyestuffs and also as intermediates of agrichemicals, pharmaceuticals, and the like.

### Description of the Background Art:

Conventional processes for the manufacture of 2-phenyl-4,5-oxazolinedione 4-phenylhydrazone derivatives comprise 3 steps; (i) a step for preparing a derivative of benzenediazonium salt by the diazotization reaction of an aniline derivative, (ii) a step for preparing a derivative of 2-phenyl-5(4H)-oxazolone from a mixed solution of a hippuric acid derivative and acetic anhydride, and (iii) a step for manufacturing a hydrazone derivative by the diazo coupling reaction of the derivative of benzenediazonium salt and the derivative of 2-phenyl-5(4H)-oxazolone. Typical processes are disclosed, for example, in EP-A-282303 (USP 4,973,353) and EP-A-310555. Reactions in these processes proceed as follows:

### Process of EP-A-282303 (USP 4,973,353)

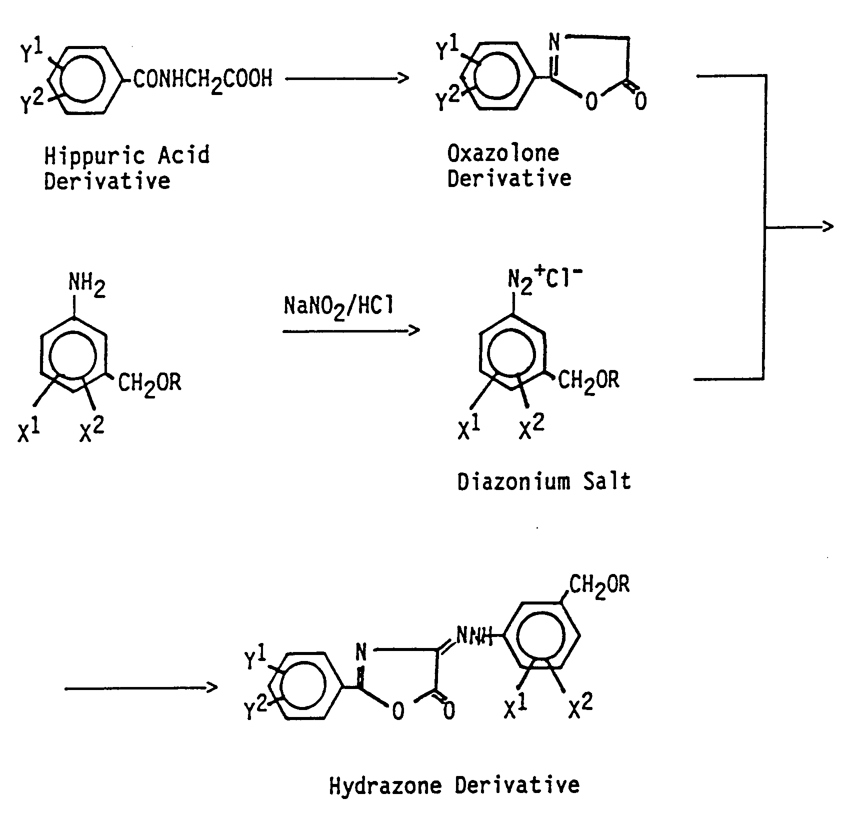

In the above process scheme, R represents a C₁-C₁₀ linear alkyl group which may be unsubstituted or substituted by fluorine, a C₃-C₁₀ branched alkyl group which may be unsubstituted or substituted by fluorine, a cyclic alkyl group, an alkyl group with an alicyclic structure, a phenyl group, or a C₇-C₉ aralkyl group; X¹ is a halogen or a C₁-C₃ alkyl group; X² is a hydrogen, a halogen, or a C₁-C₃ alkyl group; Y¹ is a hydrogen or a fluorine; and Y² is a hydrogen or a fluorine.

### Process of EP-A-310555

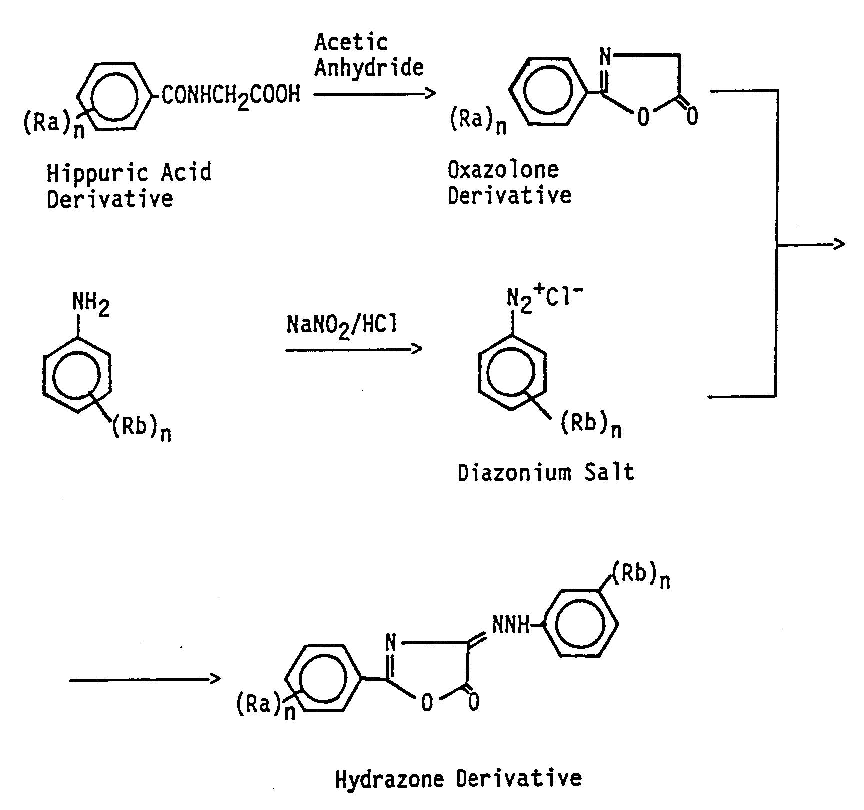

In the above process scheme, Rₐ and R_{b} each individually represents a halogen, a C₁-C₅ alkyl group, a C₁-C₅ haloalkyl group, a C₂-C₅ alkenyl group, a C₂-C₅ alkynyl group, a C₁-C₅ alkoxy group, a C₁-C₅ haloalkoxy group, a nitro group, or a cyano group; and n is an integer of 0-3.

Other prior documents frequently referred to for the manufacture of 2-phenyl-4,5-oxazolinedione 4-phenylhydrazone derivatives are Chem. Abst., 45, 7565e (1951) and J. Am. Chem. Soc., 79, 1955-1956 (1957). Regarding the step of preparing a solution of hippuric acid and acetic anhydride, the former describes a procedure of rapidly quenching a hippuric acid solution in hot acetic anhydride, and the latter proposes heating hippuric acid in acetic anhydride until a transparent solution is obtained.

Org. Syn. Coll., 5, 946-948 (1973), describes that hippuric acid rapidly converted into 2-phenyl-5(4H)-oxazolone under these conditions.

As is apparent from this prior art, the step of preparating a solution of hippuric acid and acetic anhydride, i.e. the production of an oxazolone derivative, has been considered to be indispensable for manufacturing a 2-phenyl-4,5-oxazolinedione 4-phenylhydrazone derivative.

In the step for preparing a solution of hippuric acid and acetic anhydride, which is independent from the step for preparing a derivative of diazonium salt in conventional processes, a heated solution of oxazolone derivative must be cooled to the temperature of the diazotization reaction mixture which contains neutralized diazonium derivative. Since 2-phenyl-5(4H)-oxazolone, for example, is unstable in hot acetic anhydride [Org. Syn. Coll., 5, 946-948 (1973)], the shorter is the time for the heating and cooling, the smaller is the amount of oxazolone derivative which decomposes in the reaction.

A longer period for cooling the heated solution containing oxazolone derivative thus results in a decreased yield of 4-phenylhydrazone derivative. The cooling procedure therefore must be carried out as quickly as possible in conventional processes. In mass production of 4-phenylhydrazone derivatives, in which great amounts of chemicals are involved in the preparation of the solution of hippuric acid and acetic anhydride, the rapid heating and quenching require complicated and difficult operations.

The inventors have found that reaction of (i) benzenediazonium salt compound, (ii) hippuric acid compound, and (iii) acetic anhydride in the presence of neutralizing agent (whilst separate reaction of (ii) and (iii) alone to give intermediate before reaction with (i)) can avoid the above problems in manufacturing 2-phenyl-4,5-oxazolinedione 4-phenylhydrazone compounds.

In preferred embodiments of the present invention, said neutralizing agent is one or more compounds selected from alkali metal salts of weak acids, alkaline earth metal salts of weak acids, zinc salts of weak acids, oxides of alkaline earth metals, zinc oxide, organic tertiary amines, pyridine, and pyridine derivatives.

### DETAILED DESCRIPTION OF THE INVENTION AND PREFERRED EMBODIMENTS

Derivatives of benzenediazonium salt with any substituent group(s) on the benzene ring can be used in the process of the present invention so long as such substitution does not adversely affect the diazotization reaction of the aniline derivative which is a precursor of the benzenediazonium salt. Also, any hippuric acid derivatives can be used so long as any substitution on the benzene ring does not interfere with oxazolone cyclization reaction following said diazotization reaction and the succeeding diazo coupling reaction (such cyclization reaction and diazo coupling reaction are hereinafter collectively referred to as "cyclization-coupling reaction"). Allowable substituents are as set out below for compounds (I), (II) and (III).

A preferred embodiment of the present invention is as follows: 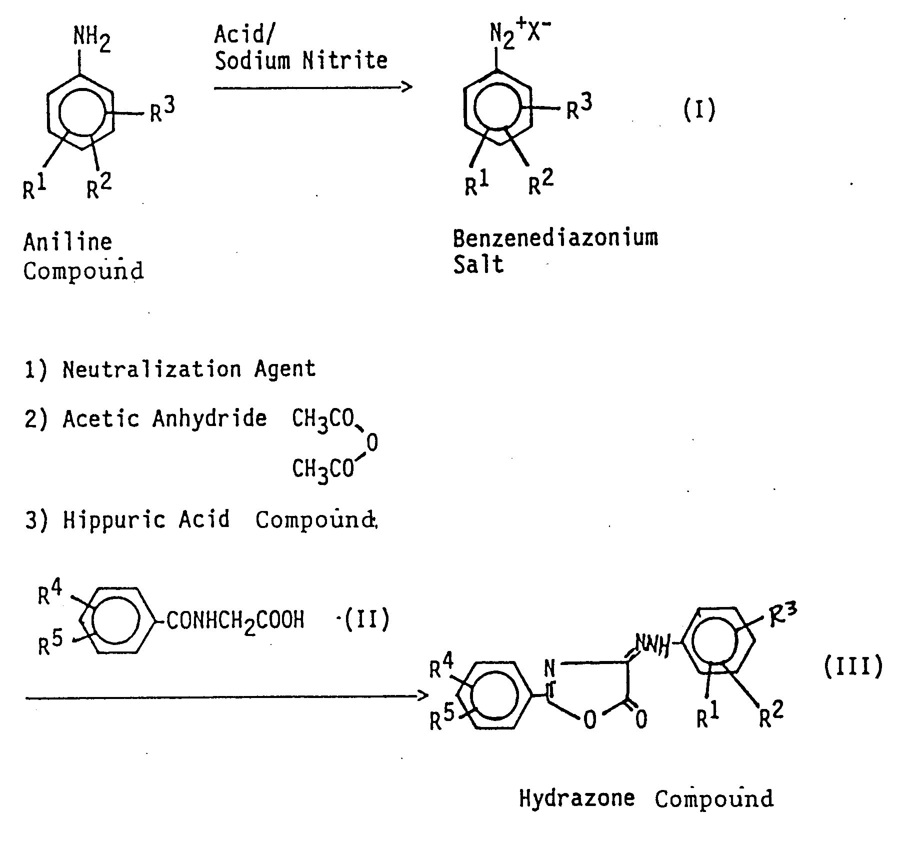 wherein R¹ is a hydrogen, a halogen, a lower alkyl group, a lower alkoxy group, a lower alkylthio group, a (lower alkoxy)carbonyl group, a lower alkyl group substituted with one or more halogen atoms, a cyano group, a nitro group, or a group AOCH₂, wherein A is a hydrogen, a substituted or unsubstituted alkyl, alkenyl, cyclopropyl/-pentyl/-hexylmethyl, aryl or aralkyl group; R² and R³ are selected independently from hydrogen, halogens, and lower alkyl groups; X⁻denotes an anion such as chloride ion, sulfate ion, tetrafluoroborate ion, or the like; R⁴ is a hydrogen, a halogen, a lower alkyl group, a lower alkoxy group, a lower alkyl group substituted with one or more halogen atoms, a nitro group, or a group A'OCH₂, wherein A' is an alkyl group substituted with one or more fluorine atoms; and R⁵ is a hydrogen, a halogen, a lower alkyl group, or a lower alkoxy group. The term "lower" above indicates preference for a carbon chain of up to 6 carbon atoms, e.g. 1, 2 or 3 carbon atoms.

According to the above reaction scheme, to benzenediazonium salt (I) (which is a diazotization product of aniline compound) are added neutralizing agent, acetic anhydride, and hippuric acid compound (II) to effect the cyclization-coupling reaction, thus producing 2-phenyl-4,5-oxazolinedione 4-phenylhydrazone compound.

For the preparation of a benzenediazonium salt, a mixture of aniline compound and inorganic acid (such as hydrochloric acid or sulfuric acid) or organic acid (such as acetic acid or propionic acid) can be reacted with stirring while an aqueous solution of nitrite, such as sodium nitrite, is added dropwise to complete the diazotization of the aniline derivative. When a surplus amount of nitrite for the amount of aniline compound is used, the addition of sulfamic acid or urea after completion of the diazotization is preferable for decomposing the unreacted nitrite.

The reaction mixture thus prepared is mixed with hippuric acid compound and acetic anhydride. In this instance, one or more inorganic or organic basic compounds are used as neutralizing agents for the benzenediazonium salt. Such neutralizing agents may be selected from alkali metal salts, alkaline earth metal salts and zinc salts of weak acids, e.g. acetate, carboxylate; alkaline earth metal oxides and zinc oxide; organic tertiary amines, e.g. triethylamine, ethyldiisopropylamine, tripropylamine, etc.; and pyridine derivatives, e.g, methylpyridine, dimethylpyridine, etc. Especially preferable among these neutralizing agents are alkali metal salts, alkaline earth metal salts,and zinc salts of weak acid, e.g., acetate, carboxylate; alkaline earth metal oxides;and zinc oxide.

There are no restrictions to the manner of the neutralization. A method of neutralizing after mixing benzenediazonium salt - e.g. (I) - hippuric acid compound - e.g. (II) - and acetic anhydride; a method of neutralizing the benzenediazonium salt , before the addition of hippuric acid compound and acetic anhydride; a method of mixing partially neutralized benzenediazonium salt with hippuric acid compound and acetic anhydride, followed by the neutralization of the remaining benzenediazonium salt; and the like are given as possible methods of neutralization. A preferable amount of neutralization agent used is 1.1-2.0 equivalents of the amount of the benzenediazonium salt.

For the control of the heat generated by the neutralization reaction, it is possible to remove heat by cooling, to add the neutralization agent in portions, or to adjust the lump size of the neutralization agent. The hydrazone compound product - e.g. (III) - can be obtained in good yield by controlling the reaction temperature below 20°C, preferably below 15°C, and especially preferably below 10°C, up to this point. Unexpectedly, heating hippuric acid compound and acetic anhydride is unnecessary.

Thereafter, the reaction temperature can be raised to 20-35°C for the cyclization-coupling reaction to obtain the target hydrazone compound. A reaction time exceeding 16 hours cannot result in a better yield of the product.

Examples of hydrazone compounds manufactured by the process of the present invention are listed in the following tables. It will be understood that herein the terms "benzediazonium salt compound", "hippuric acid compound" etc. encompass the unsubstituted materials (benzendiazonium salt, hippuric acid etc. per se) as well as substituted materials.

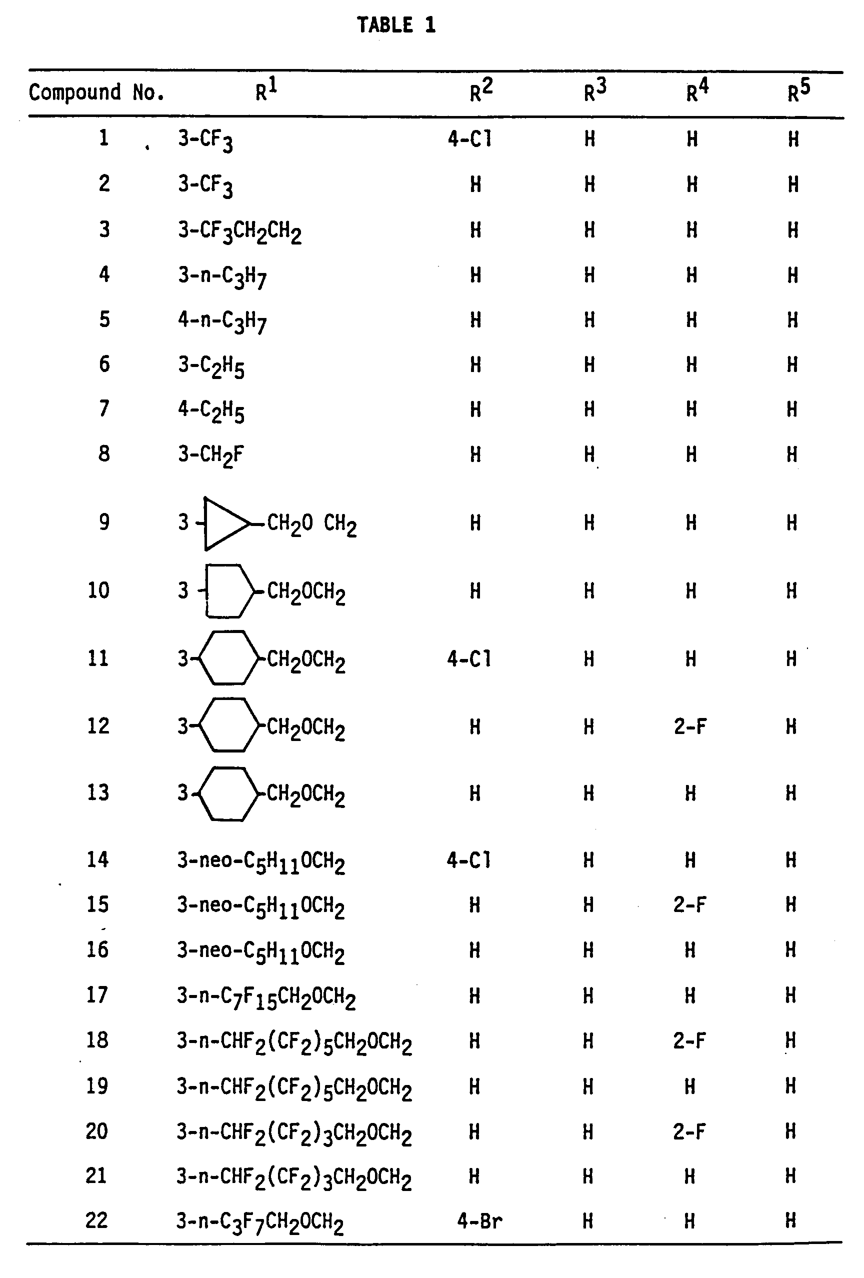

**TABLE 2**

| Compound No. | R¹ | R² | R³ | R⁴ | R⁵ |
|---|---|---|---|---|---|
| 23 | 3-n-C₃F₇CH₂OCH₂ | 4-CH₃ | H | H | H |
| 24 | 3-n-C₃F₇CH₂OCH₂ | 4-Cl | H | H | H |
| 25 | 3-n-C₃F₇CH₂OCH₂ | H | H | 4-CH₃ | H |
| 26 | 3-n-C₃F₇CH₂OCH₂ | H | H | 4-Cl | H |
| 27 | 3-n-C₃F₇CH₂OCH₂ | H | H | 2-F | H |
| 28 | 3-n-C₃F₇CH₂OCH₂ | H | H | 3-F | H |
| 29 | 3-n-C₃F₇CH₂OCH₂ | H | H | 4-F | H |
| 30 | 3-n-C₃F₇CH₂OCH₂ | H | H | H | H |
| 31 | 3-n-C₃F₇CH₂OCH₂ | H | H | 4-CH₃O | H |
| 32 | 3-C₂F₅CH₂OCH₂ | 4-Br | H | H | H |
| 33 | 3-C₂F₅CH₂OCH₂ | 4-C₂H₅ | H | H | H |
| 34 | 3-C₂F₅CH₂OCH₂ | 4-CH₃ | H | 2-F | H |
| 35 | 3-C₂F₅CH₂OCH₂ | 4-CH₃ | H | 2-F | 6-F |
| 36 | 3-CHF₂CF₂CH₂OCH₂ | 4-CH₃ | H | H | H |
| 37 | 3-C₂F₅CH₂OCH₂ | 4-CH₃ | H | H | H |
| 38 | 3-C₂F₅CH₂OCH₂ | 2-Cl | H | H | H |
| 39 | 3-C₂F₅CH₂OCH₂ | 4-Cl | H | 2-F | H |
| 40 | 3-C₂F₅CH₂OCH₂ | 4-Cl | 6-Cl | H | H |
| 41 | 3-C₂F₅CH₂OCH₂ | 4-Cl | 6-F | H | H |
| 42 | 3-C₂F₅CH₂OCH₂ | 4-Cl | H | H | H |
| 43 | 3-C₂F₅CH₂OCH₂ | 6-Cl | H | H | H |
| 44 | 3-C₂F₅CH₂OCH₂ | 4-F | H | H | H |
| 45 | 3-C₂F₅CH₂OCH₂ | H | H | 4-CH₃ | H |
| 46 | 3-C₂F₅CH₂OCH₂ | H | H | 4-Cl | H |
| 47 | 3-C₂F₅CH₂OCH₂ | H | H | 2-F | H |

**TABLE 3**

| Compound No. | R¹ | R² | R³ | R⁴ | R⁵ |
|---|---|---|---|---|---|
| 48 | 3-C₂F₅CH₂OCH₂ | H | H | 3-F | H |
| 49 | 3-C₂F₅CH₂OCH₂ | H | H | 4-F | H |
| 50 | 3-C₂F₅CH₂OCH₂ | H | H | H | H |
| 51 | 3-C₂F₅CH₂OCH₂ | 4-I | H | H | H |
| 52 | 3-CHF₂CF₂CH₂OCH₂ | 4-Cl | H | H | H |
| 53 | 3-CHF₂CF₂CH₂OCH₂ | H | H | 2-F | H |
| 54 | 3-CHF₂CF₂CH₂OCH₂ | H | H | H | H |
| 55 | 3-CF₃CHFCF₂CH₂OCH₂ | 4-CH₃ | H | H | H |
| 56 | 3-CF₃CHFCF₂CH₂OCH₂ | 4-Cl | H | H | H |
| 57 | 3-CF₃CHFCF₂CH₂OCH₂ | H | H | 2-F | H |
| 58 | 3-CF₃CHFCF₂CH₂OCH₂ | H | H | H | H |
| 59 | 3-CF₃CH₂OCH₂ | 4-Br | H | H | H |
| 60 | 3-CF₃CH₂OCH₂ | 4-CH₃ | H | H | H |
| 61 | 3-CF₃CH₂OCH₂ | 4-Cl | H | H | H |
| 62 | 3-CF₃CH₂OCH₂ | H | H | 2-F | H |
| 63 | 3-CF₃CH₂OCH₂ | H | H | H | H |
| 64 | 3-n-C₁₀H₂₁OCH₂ | H | H | H | H |
| 65 | 3-n-C₈H₁₇OCH₂ | 4-Cl | H | H | H |
| 66 | 3-n-C₈H₁₇OCH₂ | H | H | H | H |
| 67 | 3-n-C₆H₁₃OCH₂ | 4-CH₃ | H | H | H |
| 68 | 3-n-C₆H₁₃OCH₂ | 4-Cl | H | H | H |
| 69 | 3-n-C₆H₁₃OCH₂ | H | H | 2-F | H |
| 70 | 3-n-C₅H₁₁OCH₂ | 4-Cl | H | H | H |
| 71 | 3-n-C₅H₁₁OCH₂ | H | H | 2-F | H |
| 72 | 3-n-C₅H₁₁OCH₂ | H | H | H | H |

**TABLE 4**

| Compound No. | R¹ | R² | R³ | R⁴ | R⁵ |
|---|---|---|---|---|---|
| 73 | 3-n-C₇H₁₅OCH₂ | H | H | H | H |
| 74 | 3-n-C₄H₉OCH₂ | 4-Br | H | 2-F | H |
| 75 | 3-n-C₄H₉OCH₂ | 4-Br | H | H | H |
| 76 | 3-n-C₄H₉OCH₂ | 4-CH₃ | H | H | H |
| 77 | 3-n-C₄H₉OCH₂ | 4-Cl | H | 2-F | H |
| 78 | 3-n-C₄H₉OCH₂ | 4-Cl | H | H | H |
| 79 | 3-n-C₄H₉OCH₂ | H | H | 2-F | H |
| 80 | 3-n-C₆H₁₃OCH₂ | H | H | H | H |
| 81 | 3-n-C₄H₉OCH₂ | H | H | H | H |
| 82 | 3-n-C₃H₇OCH₂ | 4-CH₃ | H | H | H |
| 83 | 3-n-C₃H₇OCH₂ | H | H | H | H |
| 84 | 3-iso-C₅H₁₁OCH₂ | 4-Br | H | H | H |
| 85 | 3-iso-C₅H₁₁OCH₂ | 4-CH₃ | H | 2-F | H |
| 86 | 3-iso-C₅H₁₁OCH₂ | 4-CH₃ | H | 2-F | 6-F |
| 87 | 3-iso-C₅H₁₁OCH₂ | 4-CH₃ | H | H | H |
| 88 | 3-iso-C₅H₁₁OCH₂ | 4-Cl | H | 2-F | 6-F |
| 89 | 3-iso-C₅H₁₁OCH₂ | 4-Cl | H | 2-F | 4-F |
| 90 | 3-iso-C₅H₁₁OCH₂ | 4-Cl | H | 2-F | H |
| 91 | 3-iso-C₅H₁₁OCH₂ | 4-Cl | H | H | H |
| 92 | 3-iso-C₅H₁₁OCH₂ | 4-Cl | 6-Cl | H | H |
| 93 | 3-iso-C₅H₁₁OCH₂ | H | H | 4-CH₃ | H |
| 94 | 3-iso-C₅H₁₁OCH₂ | H | H | 4-Cl | H |
| 95 | 3-iso-C₅H₁₁OCH₂ | H | H | 2-F | H |
| 96 | 3-iso-C₅H₁₁OCH₂ | H | H | 3-F | H |
| 97 | 3-iso-C₅H₁₁OCH₂ | H | H | 4-F | H |

**TABLE 5**

| Compound No. | R¹ | R² | R³ | R⁴ | R⁵ |
|---|---|---|---|---|---|
| 98 | 3-iso-C₅H₁₁OCH₂ | H | H | H | H |
| 99 | 3-iso-C₅H₁₁OCH₂ | H | H | 4-CH₃O | H |
| 100 | 3-n-C₆H₁₃OCH₂CH₂OCH₂ | H | H | H | H |
| 101 | 3-n-C₄H₉OCH₂CH₂OCH₂ | H | H | H | H |
| 102 | 3-n-C₄H₉(OCH₂CH₂)₂OCH₂ | H | H | H | H |
| 103 | 3-C₂H₅OCH₂CH₂OCH₂ | H | H | H | H |
| 104 | 3-CH₂=CHCH₂OCH₂CH₂OCH₂ | H | H | H | H |
| 105 | 3-iso-C₄H₉OCH₂CH₂OCH₂ | H | H | H | H |
| 106 | 3-PhCH₂OCH₂CH₂OCH₂ | H | H | H | H |
| 107 | 3-CH₃OCH₂CH₂OCH₂ | H | H | H | H |
| 108 | 3-iso-C₃H₇OCH₂CH₂OCH₂ | H | H | H | H |
| 109 | 3-PhOCH₂CH₂OCH₂ | H | H | H | H |
| 110 | 3-m-CH₃PhOCH₂CH₂OCH₂ | H | H | H | H |
| 111 | 3-P-ClPhOCH₂CH₂OCH₂ | H | H | H | H |
| 112 | 3-C₂H₅OCH₂ | 4-Cl | H | H | H |
| 113 | 3-C₂H₅OCH₂ | H | H | 2-F | H |
| 114 | 3-C₂H₅OCH₂ | H | H | H | H |
| 115 | 3-CH₂=CHCH₂OCH₂ | H | H | 2-F | H |
| 116 | 3-CH₂=CHCH₂OCH₂ | H | H | H | H |
| 117 | 3-CH₃CH₂(CH₃)CHCH₂OCH₂ | 4-Cl | H | H | H |
| 118 | 3-CH₃CH₂(CH₃)CHCH₂OCH₂ | H | H | 2-F | H |
| 119 | 3-CH₃CH₂(CH₃)CHCH₂OCH₂ | H | H | H | H |
| 120 | 3-iso-C₄H₉OCH₂ | H | H | H | H |
| 121 | 3-PhCH₂OCH₂ | 4-Cl | H | H | H |
| 122 | 3-PhCH₂OCH₂ | H | H | 2-F | H |

**TABLE 6**

| Compound No. | R¹ | R² | R³ | R⁴ | R⁵ |
|---|---|---|---|---|---|
| 123 | 3-PhCH₂OCH₂ | H | H | H | H |
| 124 | 3-o-CH₃PhCH₂OCH₂ | H | H | H | H |
| 125 | 3-m-CH₃PhCH₂OCH₂ | H | H | H | H |
| 126 | 3-p-CH₃PhCH₂OCH₂ | H | H | H | H |
| 127 | 3-o-ClPhCH₂OCH₂ | H | H | H | H |
| 128 | 3-m-ClPhCH₂OCH₂ | H | H | H | H |
| 129 | 3-p-ClPhCH₂OCH₂ | H | H | H | H |
| 130 | 3-o-FPhCH₂OCH₂ | H | H | H | H |
| 131 | 3-m-FPhCH₂OCH₂ | H | H | H | H |
| 132 | 3-p-FPhCH₂OCH₂ | H | H | H | H |
| 133 | 3-m-CH₃OPhCH₂OCH₂ | H | H | H | H |
| 134 | 3-CH₃OCH₂ | H | H | H | H |
| 135 | 3-CF₃(CF₃)CHOCH₂ | H | H | H | H |
| 136 | 3-n-C₃H₇(CH₃)CHOCH₂ | H | H | H | H |
| 137 | 3-iso-C₃H₇OCH₂ | H | H | H | H |
| 138 | 3-HOCH₂ | H | H | H | H |
| 139 | 3-PhOCH₂ | 4-Cl | H | H | H |
| 140 | 3-PhOCH₂ | H | H | 2-F | H |
| 141 | 3-PhOCH₂ | H | H | H | H |
| 142 | 3-o-CF₃PhOCH₂ | H | H | H | H |
| 143 | 3-m-CF₃PhOCH₂ | H | H | H | H |
| 144 | 3-p-CF₃PhOCH₂ | H | H | H | H |
| 145 | 3-o-CH₃PhOCH₂ | H | H | H | H |
| 146 | 3-m-CH₃PhOCH₂ | H | H | H | H |
| 147 | 3-p-CH₃PhOCH₂ | H | H | H | H |

**TABLE 7**

| Compound No. | R¹ | R² | R³ | R⁴ | R⁵ |
|---|---|---|---|---|---|
| 148 | 3-p-ClPhOCH₂ | H | H | 2-F | H |
| 149 | 3-p-ClPhOCH₂ | H | H | H | H |
| 150 | 3-o-FPhOCH₂ | H | H | H | H |
| 151 | 3-m-FPhOCH₂ | H | H | H | H |
| 152 | 3-p-FPhOCH₂ | H | H | 2-F | H |
| 153 | 3-p-FPhOCH₂ | H | H | H | H |
| 154 | 3-C₆F₅OCH₂ | H | H | H | H |
| 155 | 3-p-CH₃OPhOCH₂ | H | H | H | H |
| 156 | 2-CH₃ | 3-CH₃ | 4-CH₃ | H | H |
| 157 | 2-CH₃ | 3-CH₃ | H | H | H |
| 158 | 2-CH₃ | 4-CH₃ | H | H | H |
| 159 | 2-CH₃ | 4-CH₃ | 5-CH₃ | H | H |
| 160 | 2-CH₃ | 5-CH₃ | H | H | H |
| 161 | 2-CH₃ | 6-CH₃ | H | H | H |
| 162 | 2-CH₃ | 3-Cl | H | H | H |
| 163 | 2-CH₃ | 4-Cl | H | H | H |
| 164 | 2-CH₃ | H | H | H | H |
| 165 | 3-CH₃ | 4-CH₃ | H | 4-Br | H |
| 166 | 3-CH₃ | 4-CH₃ | H | 2-CH₃ | H |
| 167 | 3-CH₃ | 4-CH₃ | H | 3-CH₃ | H |
| 168 | 3-CH₃ | 4-CH₃ | H | 3-CH₃ | 4-CH₃ |
| 169 | 3-CH₃ | 4-CH₃ | H | 4-CH₃ | H |
| 170 | 3-CH₃ | 4-CH₃ | H | 2-Cl | H |
| 171 | 3-CH₃ | 4-CH₃ | H | 3-Cl | 4-Cl |
| 172 | 3-CH₃ | 4-CH₃ | H | 3-Cl | H |

**TABLE 8**

| Compound No. | R¹ | R² | R³ | R⁴ | R⁵ |
|---|---|---|---|---|---|
| 173 | 3-CH₃ | 4-CH₃ | H | 4-Cl | H |
| 174 | 3-CH₃ | 4-CH₃ | H | 4-F | H |
| 175 | 3-CH₃ | 4-CH₃ | H | H | H |
| 176 | 3-CH₃ | 4-CH₃ | H | 4-NO₂ | H |
| 177 | 3-CH₃ | 4-CH₃ | H | 4-CH₃O | H |
| 178 | 3-CH₃ | 5-CH₃ | H | H | H |
| 179 | 3-CH₃ | 4-Cl | H | H | H |
| 180 | 3-CH₃ | 5-Cl | H | H | H |
| 181 | 3-CH₃ | 4-F | H | H | H |
| 182 | 3-CH₃ | H | H | 4-Br | H |
| 183 | 3-CH₃ | H | H | 2-CH₃ | H |
| 184 | 3-CH₃ | H | H | 3-CH₃ | H |
| 185 | 3-CH₃ | H | H | 3-CH₃ | 4-CH₃ |
| 186 | 3-CH₃ | H | H | 4-CH₃ | H |
| 187 | 3-CH₃ | H | H | 2-Cl | H |
| 188 | 3-CH₃ | H | H | 3-Cl | H |
| 189 | 3-CH₃ | H | H | 3-Cl | 4-Cl |
| 190 | 3-CH₃ | H | H | 4-Cl | H |
| 191 | 3-CH₃ | H | H | 4-F | H |
| 192 | 3-CH₃ | H | H | H | H |
| 193 | 3-CH₃ | H | H | 4-NO₂ | H |
| 194 | 3-CH₃ | H | H | 4-CH₃O | H |
| 195 | 4-CH₃ | H | H | H | H |
| 196 | 4-iso-C₃H₇ | H | H | H | H |
| 197 | 3-CN | H | H | H | H |

**TABLE 9**

| Compound No. | R¹ | R² | R³ | R⁴ | R⁵ |
|---|---|---|---|---|---|
| 198 | 3-COOCH₃ | H | H | H | H |
| 199 | 3-COO-iso-C₃H₇ | H | H | H | H |
| 200 | 2-Cl | 3-CH₃ | H | H | H |
| 201 | 2-Cl | 4-CH₃ | H | H | H |
| 202 | 2-Cl | 3-Cl | H | H | H |
| 203 | 2-Cl | 4-Cl | H | H | H |
| 204 | 2-Cl | H | H | H | H |
| 205 | 3-Cl | 4-CH₃ | H | H | H |
| 206 | 3-Cl | 4-Cl | H | H | H |

**TABLE 10**

| Compound No. | R¹ | R² | R³ | R⁴ | R⁵ |
|---|---|---|---|---|---|
| 207 | 3-Cl | 5-Cl | H | H | H |
| 208 | 3-Cl | H | H | H | H |
| 209 | 4-Cl | H | H | H | H |
| 210 | 2-F | H | H | 3-CHF₂CF₂CH₂OCH₂ | H |
| 211 | 3-F | H | H | H | H |
| 212 | 4-F | H | H | H | H |
| 213 | H | H | H | 4-Br | H |
| 214 | H | H | H | 3-CH₂F | H |
| 215 | H | H | H | 3-n-C₃F₇CH₂OCH₂ | H |
| 216 | H | H | H | 3-C₂F₅CH₂OCH₂ | H |
| 217 | H | H | H | 3-CHF₂CF₂CH₂OCH₂ | H |
| 218 | H | H | H | 3-CF₃CHFCF₂CH₂OCH₂ | H |
| 219 | H | H | H | 2-CH₃ | H |
| 220 | H | H | H | 3-CH₃ | H |
| 221 | H | H | H | 3-CH₃ | 4-CH₃ |
| 222 | H | H | H | 3-CH₃ | H |
| 223 | H | H | H | 4-CH₃ | H |
| 224 | H | H | H | 2-Cl | H |
| 225 | H | H | H | 3-Cl | H |
| 226 | H | H | H | 3-Cl | 4-Cl |
| 227 | H | H | H | 4-Cl | H |
| 228 | H | H | H | 4-F | H |
| 229 | H | H | H | H | H |
| 230 | H | H | H | 4-NO₂ | H |
| 231 | H | H | H | 4-CH₃O | H |
| 232 | 4-I | H | H | H | H |
| 233 | 4-NO₂ | H | H | H | H |
| 234 | 2-CH₃O | 5-Cl | H | H | H |
| 235 | 4-CH₃O | H | H | H | H |
| 236 | 3-CH₃S | H | H | H | H |

Other features of the invention will become apparent in the course of the following description of the exemplary embodiments which are given for illustration of the invention and are not intended to be limiting thereof.

### EXAMPLES

### Example 1

### Preparation of 2-phenyl-4,5-oxazolinedione 4-[4-chloro-3-(2,2,3,3,3-pentafluoropropoxymethyl)phenylhydrazone]

2,661 gm (9.19 mol) of 4-chloro-3-(2,2,3,3,3-pentafluoropropoxymethyl)aniline, and 4,550 ml of acetic acid, 2,110 ml of concentrated hydrochloric acid were charged into a 50 l glass-lining reaction vessel. A solution of 634 gm (9.19 mol) of sodium nitrite in 930 ml of water was added dropwise to the mixture while stirring under cooling so as not to raise the temperature above 10°C. After the addition, the mixture was stirred for 15 minutes.

To the resulting reaction mixture was added 1,578 gm of anhydrous sodium acetate while maintaining the temperature below 10°C, followed by the further addition of 1,729 gm (9.65 mol) of hippuric acid and 8,656 gm of acetic anhydride, in this order. After the addition, the mixture was heated to 30°C and stirred for 16 hours.

After the addition of 6,200 ml of water, the reaction mixture was filtered to collect 2-phenyl-4,5-oxazoledione 4-[4-chloro-3-(2,2,3,3,3-pentafluoropropoxymethyl)phenylhydrazone], which was washed with water and toluene, and dried to obtain 3,660 gm of the product. The purity of the product measured by HPLC was 98.5% and the yield corrected for purity was 85.0%.

### Example 2

### Preparation of 2-phenyl-4,5-oxazolinedione 4-[4-chloro-3-(2,2,3,3,3-pentafluoropropoxymethyl)phenylhydrazone]

29 gm (0.1 mol) of 4-chloro-3-(2,2,3,3,3-pentafluoropropoxymethyl)aniline, 50 ml of acetic acid, and 23 ml (0. 26 mol) of concentrated hydrochloric acid were charged into a 500 ml four-necked flask. A solution of 7.24 gm (0.105 mol) of sodium nitrite in 10 ml of water was added dropwise to the mixture while stirring under ice-cooling so as not to raise the temperature above 10°C. After the addition of sodium nitrite, the mixture was stirred for 20 minutes, followed by the addition of a solution of 0.74 gm of sulfamic acid (0.075 equivalent to sodium nitrite) in 7 ml of water to decompose a surplus amount of sodium nitrite in the mixture.

To the acidic solution containing a diazonium salt was added 15.09 gm (0.184 mol) of sodium acetate, by portions, followed by a further addition of 18.8 gm (0.105 mol) of hippuric acid and 93.05 gm of acetic anhydride. After the addition, the mixture was heated to 20°C and stirred for 16 hours.

After the addition of 180 ml of water, the mixture was stirred for 15 minutes to collect the precipitate by filtration. The cake thus obtained was washed with water and toluene in turn, and dried to obtain 39.58 gm of the title compound. The purity of the product was 98.1% and the yield corrected for purity was 84.1%. The yield calculated by combining 2.64 gm of the title compound contained in the toluene washing was 89.8%.

### Example 3

### Preparation of 2-phenyl-4,5-oxazolinedione 4-[4-chloro-3-(2,2,3,3,3-pentafluoropropoxymethyl)phenylhydrazone]

29 gm (0.1 mol) of 4-chloro-3-(2,2,3,3,3-pentafluoropropoxymethyl)aniline, 50 ml of acetic acid, and 23 ml (0.26 mol) of concentrated hydrochloric acid were charged into a 500 ml four-necked flask. A solution of 7.24 gm (0.105 mol) of sodium nitrite in 10 ml of water was added dropwise to the mixture while stirring under ice-cooling so as not to raise the temperature above 10°C. After the addition of sodium nitrite, the mixture was stirred for 15 minutes, followed by the addition of a solution of 0.49 gm of sulfamic acid (0.05 equivalent to sodium nitrite) in 3.5 ml of water to decompose a surplus amount of sodium nitrite in the mixture.

To the acidic solution containing a diazonium salt was added 13.8 gm (0.168 mol) of sodium acetate, by portions, followed by the further addition of 18.8 gm (0.105 mol) of hippuric acid and 93.05 gm of acetic anhydride. After the addition of acetic anhydride, the mixture was heated to 30°C and stirred for 16 hours.

After the addition of 140 ml of water, the mixture was stirred for 15 minutes to collect the precipitate by filtration. The cake thus obtained was washed with water and toluene in turn, and dried to obtain 38.85 gm of the title compound. The purity of the product was 97.9% and the yield corrected for purity was 82.5%. The yield calculated by combining 2.56 gm of the title compound contained in the toluene washing was 87.9%.

### Example 4

### Preparation of 2-phenyl-4,5-oxazolinedione 4-[4-chloro-3-(2,2,3,3,3-pentafluoropropoxymethyl)phenylhydrazone]

29 gm (0.1 mol) of 4-chloro-3-(2,2,3,3,3-pentafluoropropoxymethyl)aniline, 50 ml of acetic acid, and 23 ml (0.26 mol) of concentrated hydrochloric acid were charged into a 500 ml four-necked flask. A solution of 7.24 gm (0.105 mol) of sodium nitrite in 10 ml of water was added dropwise to the mixture while stirring under ice-cooling so as not to raise the temperature above 10°C. After the addition of sodium nitrite, the mixture was stirred for 15 minutes, followed by the addition of a solution of 0.49 gm of sulfamic acid (0.05 equivalent to sodium nitrite) in 3.5 ml of water to decompose a surplus amount of sodium nitrite in the mixture.

To the acidic solution containing a diazonium salt was added 15.09 gm (0.184 mol) of sodium acetate, by portions, followed by the further addition of 18.8 gm (0.105 mol) of hippuric acid and 17.31 gm of acetic anhydride. After the addition of acetic anhydride, the mixture was heated to 25°C and 75.74 gm of acetic anhydride was further added, followed by stirring for 16 hours.

After the addition of 180 ml of water, the mixture was stirred for 15 minutes to collect the precipitate by filtration. The cake thus obtained was washed with water and toluene in turn, and dried to obtain 39.2 gm of the title compound. The purity of the product was 97.1% and the yield corrected for purity was 82.7%. The yield calculated by combining 2.39 gm of the title compound contained in the toluene washing was 87.6%.

### Example 5

### Preparation of 2-phenyl-4,5-oxazolinedione 4-[4-chloro-3-(2,2,3,3,3-pentafluoropropoxymethyl)phenylhydrazone]

29 gm (0.1 mol) of 4-chloro-3-(2,2,3,3,3-pentafluoropropoxymethyl)aniline, 50 ml of acetic acid, and 23 ml (0.26 mol) of concentrated hydrochloric acid were charged into a 500 ml four-necked flask. A solution of 7.24 gm (0.105 mol) of sodium nitrite in 10 ml of water was added dropwise to the mixture while stirring under ice-cooling so as not to raise the temperature above 10°C. After the addition of sodium nitrite, the mixture was stirred for 15 minutes, followed by the addition of 0.6 gm of urea (0.1 equivalent to sodium nitrite) to decompose a surplus amount of sodium nitrite in the mixture.

To the acidic solution containing a diazonium salt was added 15.09 gm (0.184 mol) of sodium acetate, by portions, followed by the further addition of 18.8 gm (0.105 mol) of hippuric acid and 30 ml of acetic anhydride. After that, 20 ml, 20 ml, and 16 ml of acetic anhydride were added at an interval of 10 minutes. After the addition, the mixture was heated to 35°C for 6 hours. After terminating the heating, the mixture was further reacted for 10 hours.

After the addition of 180 ml of water, the mixture was stirred for 15 minutes to collect the precipitate by filtration. The cake thus obtained was washed with water and toluene in turn, and dried to obtain 40.2 gm of the title compound. The purity of the product was 97.0% and the yield corrected for purity was 84.6%. The yield calculated by combining 1.00 gm of the title compound contained in the toluene washing was 86.6%.

### Example 6

### Preparation of 2-phenyl-4,5-oxazolinedione 4-phenylhydrazone

9.3 gm (0.1 mol) of aniline, 50 ml of acetic acid, and 23 ml (0.26 mol) of concentrated hydrochloric acid were charged into a 500 ml four-necked flask. A solution of 7.24 gm (0.105 mol) of sodium nitrite in 10 ml of water was added dropwise to the mixture while stirring under ice-cooling so as not to raise the temperature above 5°C. After the addition of sodium nitrite, the mixture was stirred for 20 minutes.

To the acidic solution containing a diazonium salt was added 15.09gm (0.184 mol) of sodium acetate, by portions, followed by the further addition of 18.8 gm (0.105 mol) of hippuric acid and 93.05 gm of acetic anhydride. After the addition of acetic anhydride, the mixture was heated to 25°C and further reacted at this temperature for 16 hours.

After the addition of 200 ml of water, the mixture was stirred for 15 minutes to collect the precipitate by filtration. The cake thus obtained was washed 3 times with 150 ml of water, and dried to obtain 18.83 gm of the title compound. The purity of the product was 98.8% and the yield corrected for purity was 70.2%. mp: 200-203°C.

### Example 7

### Preparation of 2-phenyl-4,5-oxazolinedione 4-(m-tolylhydrazone)

10.7 gm (0.1 mol) of m-toluidine, 50 ml of acetic acid, and 23 ml (0.26 mol) of concentrated hydrochloric acid were charged into a 500 ml four-necked flask. A solution of 7.24 gm (0.105 mol) of sodium nitrite in 10 ml of water was added dropwise to the mixture while stirring under ice-cooling so as not to raise the temperature above 5°C. After the addition of sodium nitrite, the mixture was stirred for 20 minutes.

To the acidic solution containing a diazonium salt was added 15.09gm (0.184 mol) of sodium acetate, by portions, followed by the further addition of 18.8 gm (0.105 mol) of hippuric acid and 93.05 gm of acetic anhydride. After the addition of acetic anhydride, the mixture was heated to 25°C and further reacted at this temperature for 16 hours.

After the addition of 200 ml of water, the mixture was stirred for 15 minutes to collect the precipitate by filtration. The cake thus obtained was washed 3 times with 200 ml of water, and dried to obtain 19.3 gm of the title compound. The purity of the product was 99.7% and the yield corrected for purity was 68.9%. mp: 194-195°C.

### Comparative Example 1

### Preparation of 2-phenyl-4,5-oxazolinedione 4-[4-chloro-3-(2,2,3,3,3-pentafluoropropoxymethyl)phenylhydrazone]

### A. Preparation of diazonium salt

29 gm (0.1 mol) of 4-chloro-3-(2,2,3,3,3-pentafluoropropoxymethyl)aniline, 50 ml of acetic acid, and 23 ml (0.26 mol) of concentrated hydrochloric acid were charged into a 500 ml beaker and ice-cooled. A solution of 7.11 gm (0.105 mol) of sodium nitrite in 15 ml of water was added dropwise to the mixture while stirring so as not to raise the temperature above 5°C. After the addition of sodium nitrite, the mixture was stirred for 20 minutes.

### B. Preparation of 2-phenyl-5(4H)-oxazolone

23.3 gm (0.13 mol) of hippuric acid, 93.05 gm of acetic anhydride, and 50 ml of toluene were charged into a 500 ml eggplant type flask, and heated on an oil bath at a temperature of 90°C for 10 minutes to dissolution, followed by immediate quenching the resultant solution. The produced liquid was kept at -20°C on a dry ice-methanol bath.

### C. Preparation of diazonium salt and diazo coupling

To the diazonium salt which had been previously prepared was added a solution of 0.49 gm of sulfamic acid in 3.5 ml of water, followed by an addition of 13.8 gm (0.168 mol) of anhydrous sodium acetate, by portions. To the diazonium mixture was added at one time 2-phenyl-5(4H)-oxazolone which had been separately prepared. The temperature went down to -5°C, at which temperature the reaction was carried out for 4 hours. After terminating the cooling, the mixture was stirred at 20°C for 12 hours.

After the addition of 140 ml of water, the reaction mixture was stirred for 10 minutes to collect the precipitate by filtration. The cake thus obtained was washed with water and toluene in turn, and dried to obtain 38.85 gm of 2-phenyl-4,5-oxazolinedione 4-[4-chloro-3-(2,2,3,3,3-pentafluoropropoxymethyl)phenylhydrazone] which is the target compound (purity: 98.4%) in a yield of 82.9%. The yield calculated by combining 2.92 gm of hydrazone contained in the toluene washing was 89.1%.

### Comparative Example 2

### Preparation of 2-phenyl-4,5-oxazolinedione 4-[4-chloro-3-(2,2,3,3,3-pentafluoropropoxymethyl)phenylhydrazone]

### A. Preparation of 2-phenyl-5(4H)-oxazolone

23.3 gm of hippuric acid and 93.05 gm of acetic anhydride were charged into a 500 ml eggplant type flask, and heated on an oil bath at a temperature of 90°C for 10 minutes. Immediately after the mixture became transparent, it was quenched and kept at 1-2°C in ice- water.

### B. Preparation of diazonium salt and diazo coupling

29 gm (0.1 mol) of 4-chloro-3-(2,2,3,3,3-pentafluoropropoxymethyl)aniline and 50 ml of acetic acid were placed into a 1 l reaction vessel. To this was added 23 ml of concentrated hydrochloric acid and, while stirring, was further added dropwise 7.5 gm of sodium nitrite dissolved in 15 ml of water so as not to raise the temperature above 5°C. After the addition of sodium nitrite, the mixture was stirred for 20 minutes, followed by the addition of 17.2 gm of anhydrous sodium acetate and the 2-phenyl-5(4H)-oxazolone solution which had been prepared prior to 5 hours. The resulting mixture was stirred for 2 hours while controlling the temperature below 5°C, and at 20°C for 14 hours.

After the addition of 50 ml of water, the reaction mixture was filtered, washed with water and toluene in turn, and dried to obtain 20.7 gm of the title compound in a yield of 44.9%.

### Comparative Example 3

### Preparation of 2-phenyl-4,5-oxazolinedione 4-[4-chloro-3-(2,2,3,3,3-pentafluoropropoxymethyl)phenylhydrazone]

### A. Preparation diazonium salt

818 gm (2.82 mol) of an aniline derivative, 1,400 ml of acetic acid, and 648 ml (7.33 mol) of concentrated hydrochloric acid were charged into a 15 l glass lining reaction vessel and cooled to 5°C. A solution of 195 gm (2.82 mol) of sodium nitrite in 286 ml of water was added dropwise to the mixture while thoroughly stirring so as to control the temperature below 5°C.

### B. Preparation of 2-phenyl-5(4H)-oxazolone

657 gm (3.66 mol) of hippuric acid and 2,618 gm of acetic anhydride were charged into a 5 l glass reaction vessel, and heated at 80°C for 15 minutes. When the reaction liquid became transparent, the reaction vessel was placed into a water bath to lower the internal temperature to 30°C, followed by cooling to 0°C on a dry ice-methanol bath.

### C. Neutralization of diazonium salt and diazo coupling

To the diazonium salt which had been previously prepared was added 485 gm of (5.91 mol) of anhydrous sodium acetate, by portions, while controlling the temperature below 5°C. After the neutralization, the mixture was cooled to -10°C, and to this was added 2-phenyl-5(4H)-oxazolone which had been cooled to 0°C. The mixture was reacted at a temperature below 5°C for 4 hours, and at 20°C for 12 hours.

After the addition of 1,900 ml of water, the reaction mixture was stirred and filtered. The cake was washed with water and then with toluene, and dried to obtain 997 gm of the title compound. The purity determined by HPLC was 97.9%. The yield as converted 100% purity compound was 75.0%.

The raw materials, the reaction conditions, etc., are summarized in Tables 11 and 12.

**TABLE 11**

| MANUFACTURE OF HYDRAZONE DERIVATIVES - RAW MATERIALS AND NEUTRALIZATION AGENT, AND THEIR AMOUNTS USED | | | | |
|---|---|---|---|---|
| Example | Aniline Derivative gm (mol) | Hippuric Acid * Derivative gm (mol) | Acetic Anhydride (g) | Sodium Acetate gm (mol) |
| Example | | | | |
| 1 | 2,661 (9.19) | 1,729 (9.65) | 8,656 | 1,578 (91.24) |
| 2 | 29 (0.1) | 18.8 (0.105) | 93.05 | 15.09 (0.184) |
| 3 | 29 (0.1) | 18.8 (0.105) | 93.05 | 13.8 (0.168) |
| 4 | 29 (0.1) | 18.8 (0.105) | 93.05 | 15.09 (0.184) |
| 5 | 29 (0.1) | 18.8 (0.105) | 93.05 | 15.09 (0.184) |
| 6 | 9.3 (0.1) | 18.8 (0.105) | 93.05 | 15.09 (0.184) |
| 7 | 10.7 (0.1) | 18.8 (0.105) | 93.05 | 15.09 (0.184) |

| Comparative Example | | | | |
|---|---|---|---|---|
| 1 | 29 (0.1) | 23.03 (0.13) | 93.05 | 13.8 (0.168) |
| 2 | 29 (0.1) | 23.03 (0.13) | 93.05 | 17.2 (0.210) |
| 3 | 818 (2.82) | 657 (3.66) | 2,618 | 485 (5.91) |

| | | | | |
|---|---|---|---|---|
| * The hippuric acid derivative was used 1.05 equivalent to the aniline derivative in Examples 1-7, but 1.3 equivalent in Comparative Examples 1-3. | | | | |

To the aniline derivative, 1.05 equivalent of hippuric acid was used in Examples 1-7, and 1.3 equivalent of hippuric acid was used in comparative Examples 1-3.

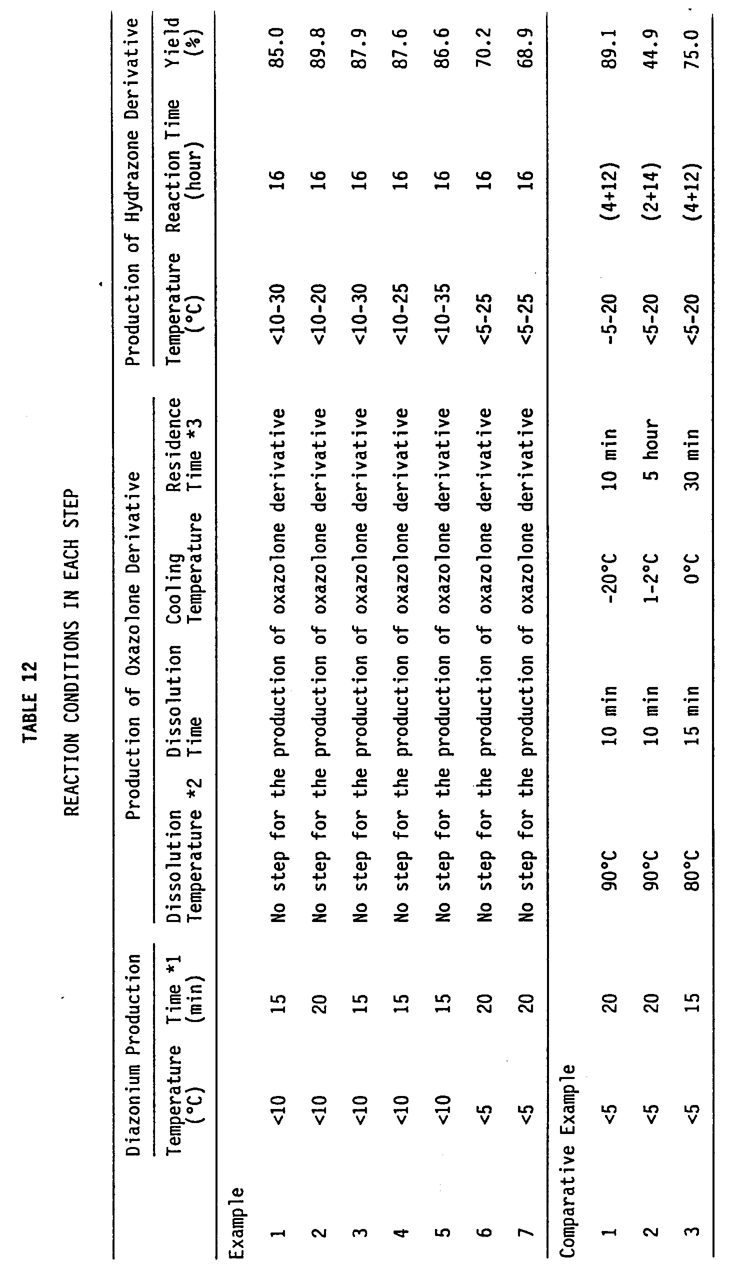

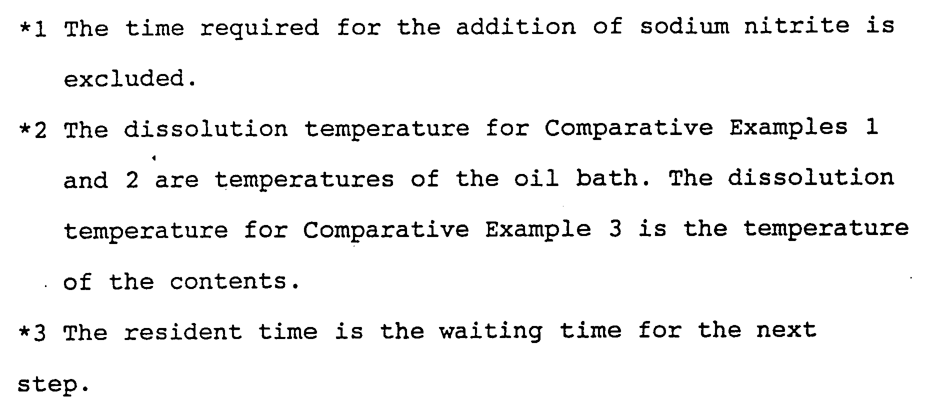

The following Examples illustrate the preparation of 2-phenyl-4,5-oxazolinedione 4-[4-chloro-3-(2,2,3,3,3-pentafluoropropoxymethyl)phenylhydrazone] (hereinafter referred to as the target compound), in which various basic compounds were used for the neutralization.

### Example 8

20 gm (0.069 mol) of 4-chloro-3-(2,2,3,3,3-pentafluoropropoxymethyl)aniline, 35.74 gm of acetic acid, and 14.40 gm (0.138 mol) of concentrated hydrochloric acid were charged into a four-necked flask and cooled to 0°C. A solution of 5.00 gm (0.069x1.05 mol) of sodium nitrite in 6.88 gm of water was added to the mixture in 15 minutes while controlling the temperature of the reaction mixture at 0-5°C. After the addition of sodium nitrite, the mixture was stirred for a further 10 minutes to obtain an acidic solution containing diazonium salt.

To the acidic solution containing a diazonium salt thus obtained were added at 20°C 14.22 gm (0.069x1.15 mol) of hippuric acid and 42.28 gm (0.069x6.00 mol) of acetic anhydride, and then 3.88 gm (0.069x1.00 mol) of pulverized calcium oxide. After the addition, the heat of neutralization was removed by cooling on a water bath to maintain the temperature at 30±1°C. After the heat removal was no longer necessary, the mixture was heated at 30±1°C.

Four hours after the addition of calcium oxide, 200 ml of water was added, and the mixture was cooled to 10°C and stirred for 30 minutes. Yellow precipitate was collected by filtration, washed 3 times with water, and dried at 80°C for 15 minutes to obtain 31.67 gm (purity: 98.74%) of the target compound. The yield corrected for purity was 98.16%.

### Example 9

To the acidic solution containing a diazonium salt prepared from 10 gm (0.0345 mol) of 4-chloro-3-(2,2,3,3,3-pentafluoropropoxymethyl)aniline in the same manner as in Example 8 were added 7.11 gm (0.0345x1.15 mol) of hippuric acid and 5.285 gm (0.0345x1.5 mol) of acetic anhydride, and the mixture was cooled to 10°C. To this was added 0.485 gm (0.0345x0.25 mol) of pulverized calcium oxide, followed by rapid heating to 20°C. Thereafter, the mixture was maintained at 20±1°C by cooling from time to time.

One and half hours thereafter, the reaction mixture was temporarily cooled to 15°C to add 5.285 gm of acetic anhydride and 0.485 gm of calcium oxide. This procedure of the addition of acetic anhydride and calcium oxide was repeated twice at an interval of 1.5 hours. The mixture was maintained at the same temperature for a further 1.5 hours after the last addition, following which it was heated at 30°C on an oil bath for 2 hours. The heat of neutralization was removed by cooling on a water bath to maintain the temperature at 30±1°C. After the removal of heat was no longer necessary, the mixture was heated at 30±1°C.

100 ml of water was added to the neutralized product, and the procedure of Example 8 was followed to obtain 15.63 gm of yellow solid of the target compound with a purity of 98.02%. The yield corrected for purity was 96.14%.

### Example 10

An acidic solution containing diazonium salt prepared from 10 gm (0.0345 mol) of 4-chloro-3-(2,2,3,3,3-pentafluoropropoxymethyl)aniline in the same manner as in Example 8 was divided into 4 equal portions. To one of the portions were added at 10°C 7.11 gm (0.0345x1.15 mol) of hippuric acid, 21.14 gm (0.0345x6.00 mol) of acetic anhydride, and 1.94 gm (0.0345x1.00 mol) of pulverized calcium oxide.

The whole mixture was solidified soon, upon which the remaining portions of the acidic solution containing diazonium salt were added and reacted for 2 hours at 30°C.

100 ml of water was added to the reaction mixture, and the procedure of Example 8 was followed to obtain 14.31 gm of yellow solid of the target compound with a purity of 98.09%. The yield corrected for purity was 88.14%.

### Example 11

An acidic solution containing diazonium salt prepared from 10 gm (0.0345 mol) of 4-chloro-3-(2,2,3,3,3-pentafluoropropoxymethyl)aniline was divided into 4 equal portions. To one of the portions were added at 15°C 7.11 gm (0.0345x1.15 mol) of hippuric acid, 21.14 gm (0.0345x6.00 mol) of acetic anhydride, and 0.485 gm (0.0345x0.25 mol) of pulverized calcium oxide.

One and half hours thereafter, the reaction mixture was temporarily cooled to 15°C to add 1/3 of the remaining portions of the acidic solution containing diazonium salt and 0.485 gm of calcium oxide. This procedure of the addition of acidic solution containing diazonium salt and calcium oxide was repeated twice at an interval of 1.5 hours. The mixture was maintained at the same temperature for a further 1.5 hours after the last addition, following which it was heated at 30°C on an oil bath for 2 hours. The heat of neutralization was removed in a water bath to maintain the temperature at 30±1°C. After the heat removal was no longer necessary, the mixture was heated at 30±1°C.

100 ml of water was added to the reaction mixture, and the procedure of Example 8 was followed to obtain 14.18 gm of yellow solid of the target compound with a purity of 97.81%. The yield corrected for purity was 87.06%.

### Example 12

To the acidic solution containing a diazonium salt prepared in the same manner as in Example 8 from 10 gm (0.0345 mol) of 4-chloro-3-(2,2,3,3,3-pentafluoropropoxymethyl)aniline were added at 15°C 7.11 gm (0.0345x1.15 mol) of hippuric acid and 21.14 gm (0.0345x6.00 mol) of acetic anhydride, and 0.485 gm (0.0345x0.25 mol) of pulverized calcium oxide.

Two hours thereafter, the reaction mixture was temporarily cooled to 15°C to add 0.485 gm of calcium oxide. This procedure of the addition of 0.485 gm of calcium oxide was repeated twice 2 hours and 3 hours thereafter. The mixture was maintained at the same temperature for a further 1.5 hours after the last addition, following which it was heated at 30°C on an oil bath for 2 hours. The heat of neutralization was removed by cooling on a water bath to maintain the temperature at 30±1°C. After the heat removal was no longer necessary, the mixture was heated at 30±1°C.

100 ml of water was added to the reaction mixture, and the procedure of Example 8 was followed to obtain 12.79 gm of yellow solid of the target compound with a purity of 98.10%. The yield corrected for purity was 78.78%.

### Example 13

An acidic solution containing a diazonium salt was prepared in the same manner as in Example 8 from 10 gm (0.0345 mol) of 4-chloro-3-(2,2,3,3,3-pentafluoropropoxymethyl)aniline. To this solution were added 7.11 gm (0.0397 mol) of hippuric acid and 21.14 gm (0.207 mol) of acetic anhydride, and then 1.07 gm (0.0190 mol) of pulverized calcium oxide at 20°C. The cyclization-coupling reaction was carried out at 20-30°C for 4 hours in the same manner as in Example 8 to obtain 15.93 gm of the target compound with a purity of 98.16%. The yield corrected for purity was 98.16%.

### Example 14

An acidic solution containing a diazonium salt was prepared in the same manner as in Example 8 from 10 gm (0.0345 mol) of 4-chloro-3-(2,2,3,3,3-pentafluoropropoxymethyl)aniline. To this solution were added 7.11 gm (0.0397 mol) of hippuric acid and 21.14 gm (0.207 mol) of acetic anhydride, and then 5.29 gm (0.0345 mol) of pulverized barium oxide at 20°C. The cyclization-coupling reaction was carried out at 20-30°C for 4 hours in the same manner as in Example 8 to obtain 13.33 gm of the target compound with a purity of 95.71%. The yield corrected for purity was 80.11%.

### Example 15

An acidic solution containing a diazonium salt was prepared in the same manner as in Example 8 from 10 gm (0.0345 mol) of 4-chloro-3-(2,2,3,3,3-pentafluoropropoxymethyl)aniline. To this solution were added 7.11 gm (0.0397 mol) of hippuric acid and 21.14 gm (0.207 mol) of acetic anhydride, and then 2.81 gm (0.0345 mol) of pulverized zinc oxide at 20°C. The cyclization-coupling reaction was carried out at 20-30°C for 4 hours in the same manner as in Example 8 to obtain 14.70 gm of the target compound with a purity of 97.46%. The yield corrected for purity was 89.90%.

### Example 16

An acidic solution containing a diazonium salt was prepared in the same manner as in Example 8 from 10 gm (0.0345 mol) of 4-chloro-3-(2,2,3,3,3-pentafluoropropoxymethyl)aniline. To this solution were added 7.11 gm (0.0397 mol) of hippuric acid and 21.14 gm (0.207 mol) of acetic anhydride, and then 1.39 gm (0.0345 mol) of pulverized magnesium oxide at 20°C. The cyclization-coupling reaction was carried out at 20-30°C for 4 hours in the same manner as in Example 8 to obtain 15.92 gm of the target compound with a purity of 97.56%. The yield corrected for purity was 97.47%.

### Example 17

An acidic solution containing a diazonium salt was prepared in the same manner as in Example 8 from 10 gm (0.0345 mol) of 4-chloro-3-(2,2,3,3,3-pentafluoropropoxymethyl)aniline. To this solution were added 7.11 gm (0.0397 mol) of hippuric acid and 21.14 gm (0.207 mol) of acetic anhydride, and then 6.81 gm (0.0345 mol) of pulverized barium carbonate at 20°C. The cyclization-coupling reaction was carried out at 20-30°C for 4 hours in the same manner as in Example 8 to obtain 14.18 gm of the target compound with a purity of 97.39%. The yield corrected for purity was 86.68%.

### Example 18

An acidic solution containing a diazonium salt was prepared in the same manner as in Example 8 from 10 gm (0.0345 mol) of 4-chloro-3-(2,2,3,3,3-pentafluoropropoxymethyl)aniline. To this solution were added 7.11 gm (0.0397 mol) of hippuric acid and 21.14 gm (0.207 mol) of acetic anhydride, and then 3.45 gm (0.0345 mol) of pulverized calcium carbonate at 20°C. The cyclization-coupling reaction was carried out at 20-30°C for 4 hours in the same manner as in Example 8 to obtain 15.92 gm of the target compound with a purity of 97.76%. The yield corrected for purity was 97.70%.

### Example 19

An acidic solution containing a diazonium salt was prepared in the same manner as in Example 8 from 10 gm (0.0345 mol) of 4-chloro-3-(2,2,3,3,3-pentafluoropropoxymethyl)aniline. To this solution were added 7.11 gm (0.0397 mol) of hippuric acid and 21.14 gm (0.207 mol) of acetic anhydride, and then 5.46 gm (0.0345 mol) of pulverized calcium acetate at 20°C. The cyclization-coupling reaction was carried out at 20-30°C for 4 hours in the same manner as in Example 8 to obtain 15.91 gm of the target compound with a purity of 98.10%. The yield corrected for purity was 98.00%.

### Example 20

An acidic solution containing a diazonium salt was prepared in the same manner as in Example 8 from 11.59 gm (0.04 mol) of 4-chloro-3-(2,2,3,3,3-pentafluoropropoxymethyl)aniline. To this solution were added 8.24 gm (0.046 mol) of hippuric acid and 36.75 gm (0.36 mol) of acetic anhydride, and then 8.10 gm (0.08 mol) of triethylamine at 20°C. The cyclization-coupling reaction was carried out at 15-35°C for 5 hours in the same manner as in Example 8 to obtain 16.82 gm of the target compound with a purity of 90.70%. The yield corrected for purity was 82.60%.

### Example 21

An acidic solution containing a diazonium salt was prepared in the same manner as in Example 8 from 11.59 gm (0.04 mol) of 4-chloro-3-(2,2,3,3,3-pentafluoropropoxymethyl)aniline. To this solution were added 8.24 gm (0.046 mol) of hippuric acid and 36.75 gm (0.36 mol) of acetic anhydride, and then 6.33 gm (0.08 mol) of pyridine at 20°C. The cyclization-coupling reaction was carried out at 10-32°C for 5 hours in the same manner as in Example 8 to obtain 18.59 gm of the target compound with a purity of 96.10%. The yield corrected for purity was 96.70%.

### Example 22

An acidic solution containing a diazonium salt was prepared in the same manner as in Example 8 from 11.59 gm (0.04 mol) of 4-chloro-3-(2,2,3,3,3-pentafluoropropoxymethyl)aniline. To this solution were added 8.24 gm (0.046 mol) of hippuric acid and 36.75 gm (0.36 mol) of acetic anhydride, and then 2.24 gm (0.04 mol) of calcium oxide pulverized to sizes of beans at 20°C. The cyclization-coupling reaction was carried out at 5-32°C for 5 hours in the same manner as in Example 8 to obtain 18.82 gm of the target compound with a purity of 98.70%. The yield corrected for purity was 97.70%.

The relations of neutralization agents, the cyclization-coupling reaction conditions, and the yield in Examples 1-22 Comparative Examples 1-3 are summarized in Table 13.

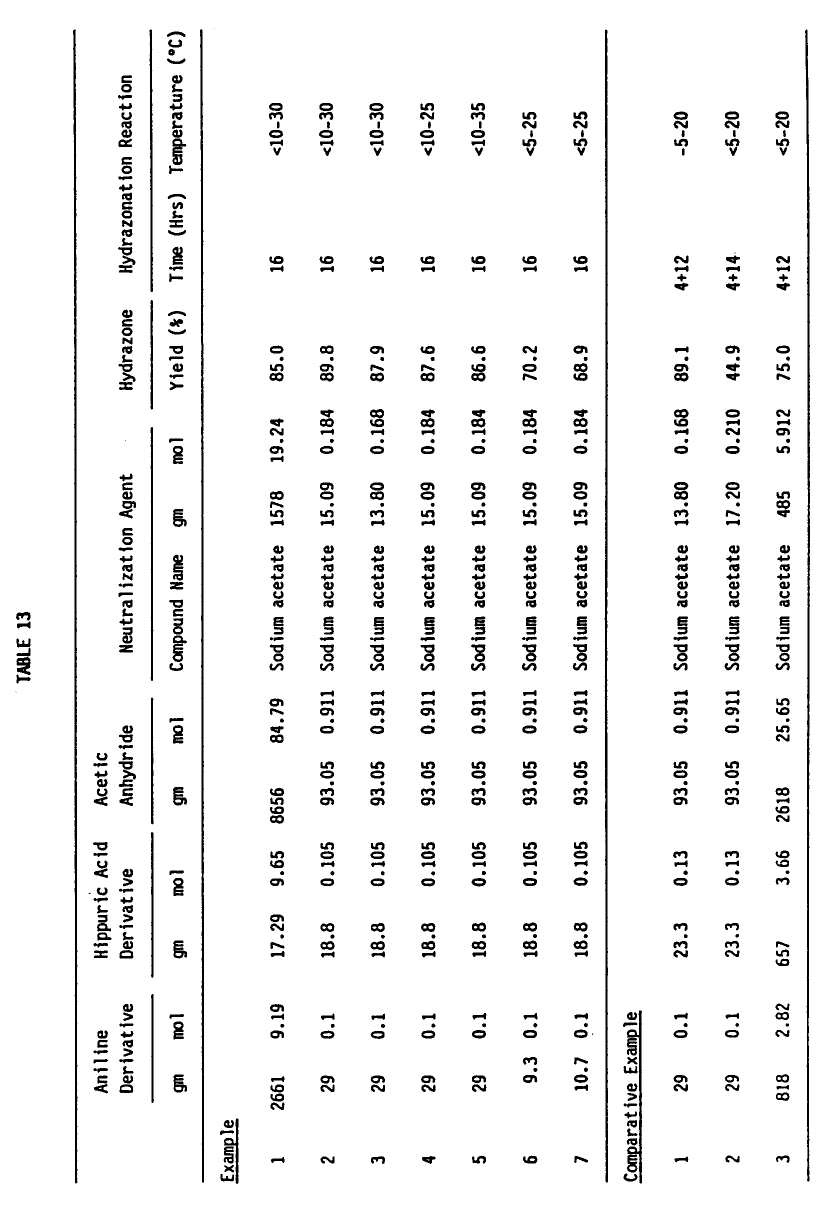

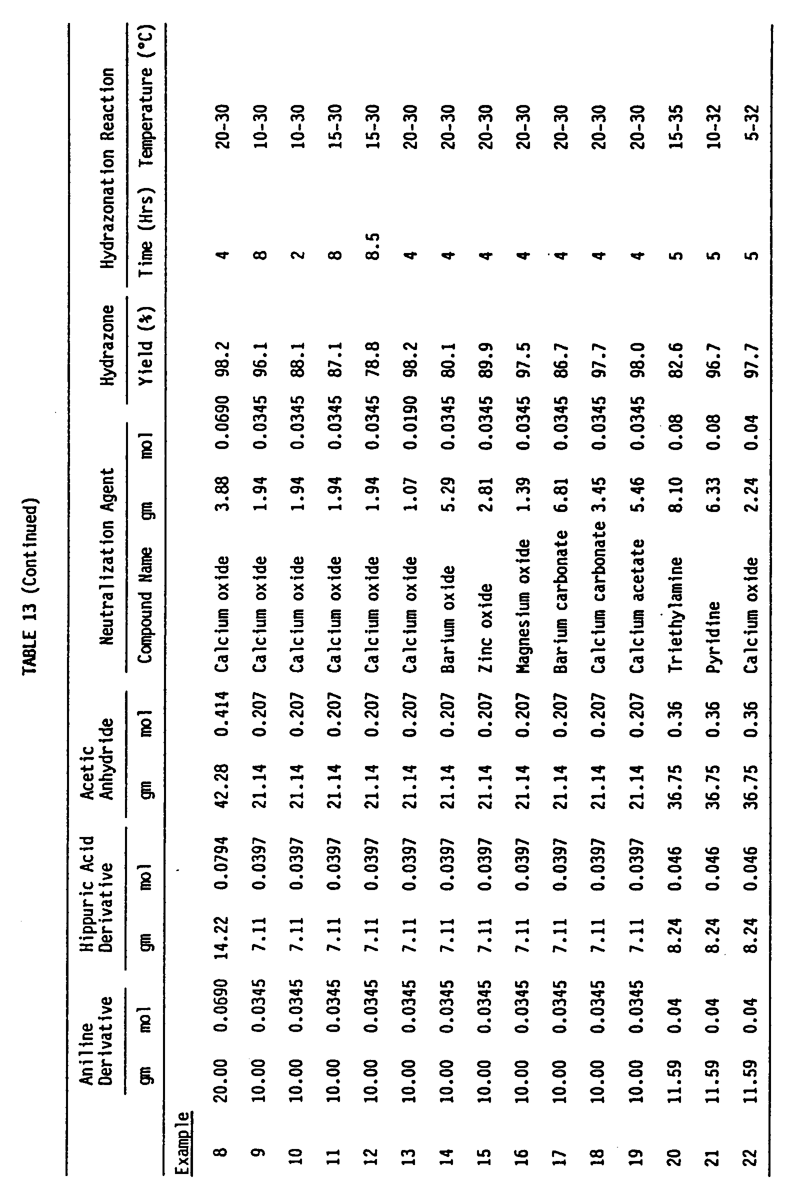

As indicated in the process scheme of the present invention and as illustrated above, the process of the present invention does not require a separate, independent step for the preparation of a solution of hippuric acid derivative and acetic anhydride. This ensures elimination of the complicated rapid heating and quenching procedures required in this step in the mass production of 2-phenyl-4,5-oxazolinedione 4-phenylhydrazone derivatives. Furthermore, the process of the present invention ensures a significant reduction in the surplus amount of hippuric acid derivatives to be used, which are required in conventional processes in order to avoid the decrease in the yield of the target compound due to decomposition of oxazolone derivatives which are cyclized in hot acetic anhydride. Thus, the process was successful in manufacturing 2-phenyl-4,5-oxazolinedione 4-phenylhydrazone derivatives in a high yield in a short period of time.

Obviously, numerous modifications and variations of the present invention are possible in light of the above teachings. It is therefore to be understood that the scope of the appended claims, the invention may be practiced otherwise than as specifically described herein.

## Claims

1. A process for manufacturing 2-phenyl-4,5-oxazolinedione 4-phenylhydrazone compound (III) which comprises reacting in the presence of neutralizing agent a mixture of (i) benzene-diazonium salt compound (I), (ii) hippuric acid compound (II) and (iii) acetic anhydride, without separate reaction of (ii) and (iii) alone to give 2-phenyl-5(4H)-oxazolone compound before reaction with (i) 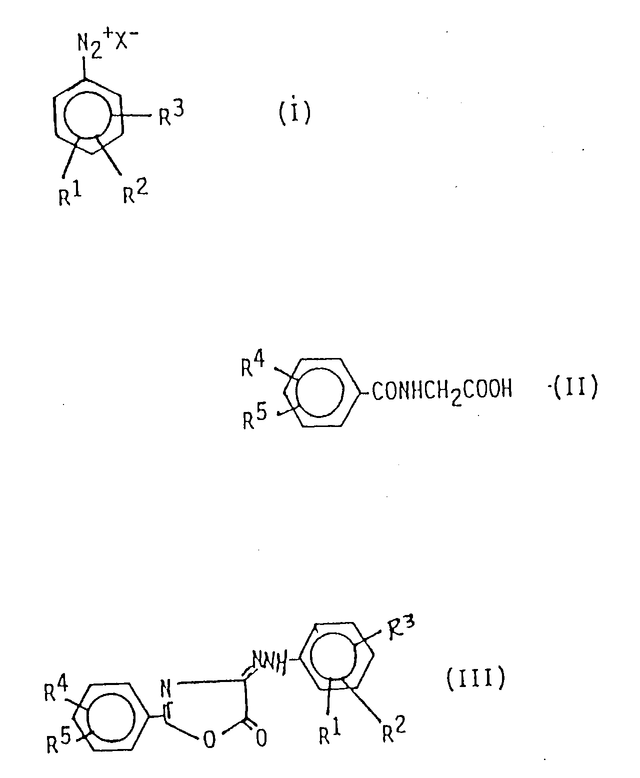 wherein R¹ is a hydrogen, a halogen, a C₁-C₆ alkyl group, a C₁-C₆ alkoxy group, a C₁-C₆ alkylthio group, a (C₁ - C₆ alkoxy)carbonyl group, a C₁-C₆ alkyl group substituted with one or more halogen atoms, a cyano group, a nitro group, or a group AOCH₂, wherein A is a hydrogen, a substituted or unsubstituted alkyl, alkenyl, cyclopropyl/-pentyl/-hexylmethyl, aryl, or aralkyl group; R² and R³ are selected independently from hydrogen, halogens, and C₁-C₆ alkyl groups; X⁻denotes an anion; R⁴ is a hydrogen, a halogen, a C₁-C₆ alkyl group, a C₁-C₆ alkoxy group, a C₁-C₆ alkyl group substituted with one or more halogen atoms, a nitro group, or a group A'OCH₂, wherein A' is an alkyl group substituted with one or more fluorine atoms; and R⁵ is a hydrogen, a halogen, a C₁-C₆ alkyl group, or a C₁-C₆ alkoxy group.

2. A process according to Claim 1 wherein said neutralizing agent is one or more compounds selected from alkali metal salts of weak acids, alkaline earth metal salts of weak acids, zinc salts of weak acids, oxides of alkaline earth metals, zinc oxide, organic tertiary amines, pyridine, and pyridine derivatives.

3. A process according to Claim 1 or 2 wherein said neutralization agent is mixed together with said benzenediazonium salt compound, hippuric acid compound, and acetic anhydride.

4. A process according to Claim 1 or 2 wherein said neutralization agent is mixed with said benzenediazonium salt compound, followed by the addition of said hippuric acid compound and acetic anhydride.

5. A process according to Claim 1 or 2 wherein a portion of said neutralization agent is mixed with said benzenediazonium salt compound followed by the addition of said hippuric acid compound, acetic anhydride, and the remaining portion of neutralization agent.

6. A process according to any preceding claim wherein the amount of said neutralization agent is 1.2-2.0 equivalents of the amount of said benzenediazonium salt compound.

7. A process according to any preceding claim wherein the mixture of said benzenediazonium salt compound, hippuric acid compound, acetic anhydride, and neutralizing agent is kept at a temperature below 20°C before reaction at 20-35°C.

8. A process according to Claim 7 wherein said mixture is kept at a temperature below 15°C before reaction at 20-35°C.

9. A process according to Claim 8 wherein said mixture is kept at a temperature below 10°C before reaction at 20-35°C.

## Patentansprüche

1. Verfahren zur Herstellung einer 2-Phenyl-4,5-oxazolindion-4-phenylhydrazon-Verbindung (III), bei dem man in Gegenwart eines Neutralisierungsmittels ein Gemisch aus
(i) einem Benzoldiazoniumsalz (I),
(ii) Hippursäure (II) und
(iii) Essigsäureanhydrid umsetzt, ohne (ii) und (iii) allein unter Bildung einer 2-Phenyl-5(4H)-oxazolon-Verbindung vor dem Umsetzen mit (i) umsetzt: 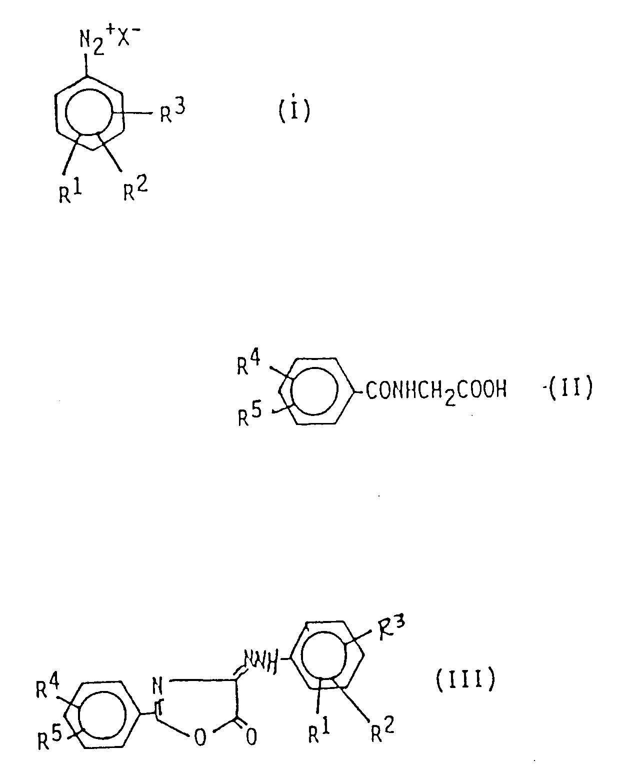 worin R¹ ein Wasserstoffatom, ein Halogenatom, eine C₁₋₆-Alkylgruppe, eine C₁₋₆-Alkoxygruppe, eine C₁₋₆-Alkylthiogruppe, eine (C₁₋₆-Alkoxy)-Carbonylgruppe, eine C₁₋₆-Alkylgruppe, die mit einem oder mehreren Halogenatomen substituiert ist, eine Cyanogruppe, eine Nitrogruppe oder eine AOCH₂-Gruppe, worin A ein Wasserstoffatom, eine substituierte oder unsubstituierte Alkyl-, Alkenyl-, Cyclopropyl-, Cyclopentyl-, Cyclohexylmethyl-, Arylgruppe oder eine Aralkylgruppe ist; R² und R³ unabhängig voneinander ein Wasserstoffatom, ein Halogenatom oder eine C₁₋₆-Alkylgruppe sind; X⁻ ein Anion bezeichnet; R⁴ ein Wasserstoffatom, ein Halogenatom, eine C₁₋₆-Alkylgruppe, eine C₁₋₆-Alkoxygruppe, eine C₁₋₆-Alkylgruppe ist, die mit einem oder mehreren Halogenatomen substituiert ist, eine Nitrogruppe oder eine A'OCH₂-Gruppe ist, worin A' eine Alkylgruppe ist, die mit einem oder mehreren Fluoratomen substituiert ist, und R⁵ ein Wasserstoffatom, eine Halogengruppe, eine C₁₋₆-Alkylgruppe oder eine C₁₋₆-Alkoxygruppe ist.

2. Verfahren nach Anspruch 1, bei dem man als Neutralisierungsmittel eine oder mehrere Verbindungen aus der durch Alkalimetallsalze schwacher Säuren, Erdalkalimetallsalze schwacher Säuren, Zinksalze schwacher Säuren, Oxide von Erdalkalimetallen, Zinkoxid, organische tertiäre Amine, Pyridin und Pyridinderivate gebildeten Gruppe verwendet.

3. Verfahren nach Anspruch 1 oder 2, bei dem man das Neutralisierungsmittel mit dem Benzoldiazoniumsalz, der Hippursäure und Essigsäureanhydrid vermischt.

4. Verfahren nach Anspruch 1 oder 2, bei dem das Neutralisierungsmittel mit dem Benzoldiazoniumsalz vermischt, wonach man die Hippursäure und Essigsäureanhydrid zugibt.

5. Verfahren nach Anspruch 1 oder 2, bei dem man einen Anteil des Neutralisierungsmittels mit dem Benzoldiazoniumsalz vermischt, wonach man die Hippursäure, Essigsäureanhydrid und den verbliebenen Anteil des Neutralisierungsmittels zugibt.

6. Verfahren nach einem der vorhergehenden Ansprüche, wobei die Menge an Neutralisierungsmittel 1,2 bis 2,0 Äquivalente der Menge an Benzoldiazoniumsalz beträgt.

7. Verfahren nach einem der vorhergehenden Ansprüche, bei dem man das Gemisch des Benzoldiazoniumsalzes, der Hippursäure, des Essigsäureanhydrids und des Neutralisierungsmittels bei einer Temperatur unter 20 °C hält, bevor man bei 20 bis 35 °C umsetzt.

8. Verfahren nach Anspruch 7, bei dem man das Gemisch bei einer Temperatur unterhalb 15 °C hält, bevor man bei 20 bis 35 °C umsetzt.

9. Verfahren nach Anspruch 8, bei dem man das Gemisch bei einer Temperatur unterhalb 10 °C hält, bevor man bei 20 bis 35 °C umsetzt.

## Revendications

1. Procédé pour la préparation d'un composé 2-phényl-4,5-oxazolinedione-4-phénylhydrazone (III) qui comporte le fait de faire réagir en présence d'un agent neutralisant un mélange de (i) un composé (I) de sel de benzène-diazonium, (ii) un composé (II) de l'acide hippurique, et de l'anhydride acétique (iii), sans réaction séparée de (ii) et de (iii) seuls, pour donner un composé de 2-phényl-5(4H)-oxazolone avant réaction avec (i) 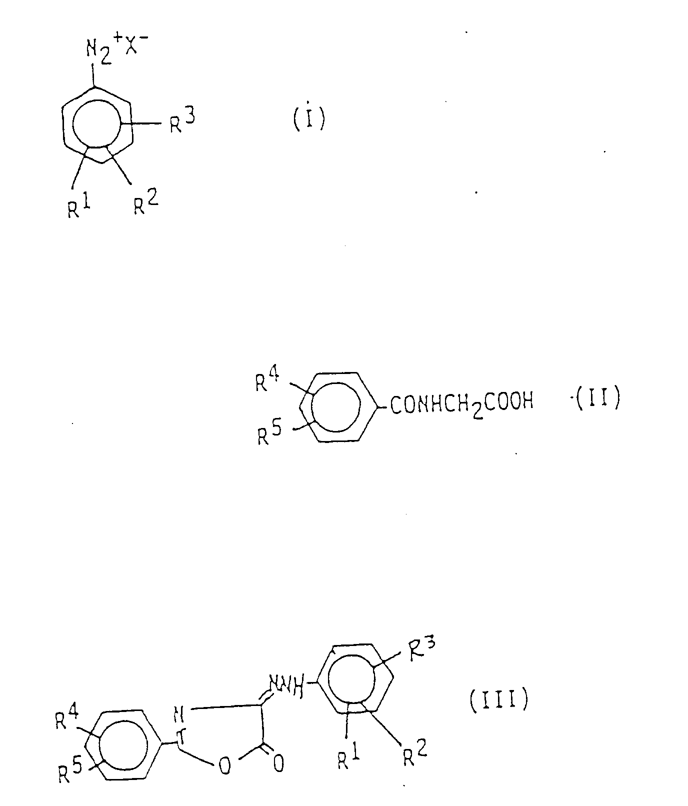 où R¹ est un hydrogène, un halogène, un groupe alkyle en C₁-C₆, un groupe alcoxy en C₁-C₆, un groupe alkylthio en C₁-C₆, un groupe(alcoxy en C₁-C₆)carbonyle, un groupe alkyle en C₁-C₆ substitué avec un ou plusieurs atomes d'halogènes, un groupe cyano, un groupe nitro, ou un groupe AOCH₂, où A est un hydrogène, un alkyle substitué ou non substitué, un alcényle, un groupe cyclopropyle, cyclopentyle, cyclohexylméthyle, aryle ou aralkyle ; R² et R³ sont choisis de façon indépendante parmi l'hydrogène, les halogènes et les groupes alkyles en C₁-C₆ ; X⁻ représente un anion ; R⁴ est un hydrogène, un halogène, un groupe alkyle en C₁-C₆, un groupe alcoxy en C₁-C₆, un groupe alkyle en C₁-C₆ substitué avec un ou plusieurs atomes d'halogènes, un groupe nitro, ou un groupe A'OCH₂, où A' est un groupe alkyle substitué avec un ou plusieurs atomes de fluor ; et R⁵ est un hydrogène, un halogène, un groupe alkyle en C₁-C₆ ou un groupe alcoxy en C₁-C₆.

2. Procédé selon la revendication 1, où ledit agent de neutralisation est un ou plusieurs composés choisis parmi les sels de métaux alcalins d'acides faibles, les sels de métaux alcalino-terreux d'acides faibles, les sels de zinc d'acides faibles, les oxydes de métaux alcalino-terreux, l'oxyde de zinc, les amines tertiaires organiques, la pyridine et les dérivés de pyridine.

3. Procédé selon la revendication 1 ou 2, où ledit agent de neutralisation est mélangé avec ledit composé de sel de benzènediazonium, un composé de l'acide hippurique, et l'anhydride acétique.

4. Procédé selon la revendication 1 ou 2, où ledit agent de neutralisation est mélangé avec ledit composé de sel de benzènediazonium, suivi de l'addition dudit composé de l'acide hippurique, et de l'anhydride acétique.

5. Procédé selon la revendication 1 ou 2, où l'on mélange une portion dudit agent de neutralisation avec ledit composé de sel de benzenediazonium suivi de l'addition dudit composé de l'acide hippurique, de l'anhydride acétique et de la portion restante de l'agent de neutralisation.

6. Procédé selon l'une quelconque des revendications précédentes, où la quantité dudit agent de neutralisation est 1,2 ou 2,0 équivalents de la quantité dudit composé de sel de benzènediazonium.

7. Procédé selon l'une quelconque des revendications précédentes où le mélange dudit composé de sel de benzènediazonium, du composé de l'acide hippurique, de l'anhydride acétique et de l'agent de neutralisation est maintenu à une température inférieure à 20°C avant la réaction à 20-35°C.

8. Procédé selon la revendication 7, où ledit mélange est maintenu à une température inférieure à 15°C avant la réaction à 20-35°C.

9. Procédé selon la revendication 8, où ledit mélange est maintenu à une température inférieure à 10°C avant la réaction à 20-35°C.
